# EUROPEAN PATENT APPLICATION

(11) **EP 1 421 930 A2**
(43) Date of publication of application: **26.05.2004**
(21) Application number: 03257362.8
(22) Date of filing: 21.11.2003
(51) Int. Cl.: A61K 7/06, A61K 7/48

(54) **Cosmetic composition, especially for dark hair**

(30) Priority: 21.11.2002 BR 0200638
(71) Applicant: Johnson & Johnson Industrial Ltda., 12237-350 Sao José dos Campos SP (BR)
(72) Inventor: Masiero, Silvana, Sao dos Campos 12245-820 SP (BR)
(74) Representative: Mercer, Christopher Paul

(57) **Abstract**

This invention relates to a cosmetic composition comprising:
(1) rosemary (Rosmarinus officinalis L. extract) - 0,001 % - 10%;
(2) henna (Lawsonia inermis L. extract) - 0,001 % - 10%;
(3) melanin - 0,000005% - 5%;
and also to compositions containing
(1) rosemary (Rosmarinus officinalis L. extract) - 0,001% - 10%;
(2) henna (Lawsonia inermis L. extract) - 0,001% - 10%;
(3) melanin - 0,000005% - 5%;
(4) sunflower (Helianthus annuss L. extract) - 0,0001% - 10%.

Combination of the above naturally-occurring components is suitable for preparing finished cosmetic compositions in association with an acceptable cosmetic base.

The compositions are particularly useful for dark hair and exhibit sun protection against UV radiation, protection of hair fiber chemical structure, protection and retention of hair color and appropriate gloss.

## Description

### Field of the Invention

The present invention relates to a cosmetic hair composition, especially for dark hair, having a low degree of eye irritation, improved gloss property and ability to retain color and protect against ultraviolet radiation. More specifically, this invention relates to compositions having a combination of certain naturally occurring components associated with a base composition usually employed in cosmetic compositions.

### Description of the Prior Art

There is a variety of cosmetic compositions in the art, for babies, kids and adults, with low eye irritation and using several types of surfactants which cause little or no eye and skin irritability. A number of surfactants with low irritability have been developed.

Another usually desirable aspect in available cosmetic compositions is their foamability. Foam is highly relevant to cleaning compositions, especially personal care products such as shampoos, liquid soap for the body, hands and face. The quality and quantity of foam have been determining factors to consumers who associate such property to product quality and efficacy. As to functionality, foam helps in emulsifying soil and provides for a more effective cleaning, loading it during the cleaning process.

Cosmetic compositions with effects of hair color retention are also available in the art. Such compositions - in addition to standard gloss-, foam-providing (usually surfactants and foaming agents) components and formulations aids - further comprise a specific dye or usual UV sunscreens (octyl methoxycinnamate, benzophenone 4, benzophenone 3) for retaining the hair natural color.

Cosmetic compositions comprising, inter alia, Henna extract are well-known compositions. GB patent 465456 and EP patent 768,865 and EP0084545 (WO 8300434) are illustrative examples of art references disclosing cosmetic compositions comprising Henna.

US 5,686,084 discloses melanin or melanin-like synthetic compounds and the use thereof as hair coloring agents or dye. Such reference shows some problems associated with the use of dyes from naturally occurring melanin due to its non-fixation ability during hair washing and loss of said ability upon rinsing. Said reference further discloses the use of melanin as dues in hair coloring compositions. Similarly, US 5,702,712 discloses similar compounds and compositions.

Compositions for hair protection against ultraviolet radiation are found in the art. Examples of compositions specially developed for such purposes can be found, inter alia, in patent application PCT/FR01/03637 (WO 02/49597), US 6,375,936 and CN 1,341,413.

### Summary of the Invention

It is the object of the present invention to provide cosmetic compositions specially developed for hair.

It is the object of the present invention to provide cosmetic compositions specially developed for hair, and in particular for dark hair, having a low degree of eye irritation and providing improved gloss, retention of hair color and protection against ultraviolet radiation.

It is another object of the present invention to provide cosmetic compositions specially developed for dark hair, having a low degree of eye irritation which, besides providing improved gloss and retention of hair color - are naturally occurring components-based.

Another aspect related to the cosmetic compositions of the present invention is that the compositions now developed are turbidity-free and clear. Use of chemical screens usually employed in conventional compositions provides turbidity (opaque product) in shampoo compositions in which they are incorporated and such drawback is overcome by the compositions of the invention.

### Detailed Description of the Invention

The cosmetic compositions of the present invention have a low degree of eye irritation and improved gloss, provide hair color retention and protection against ultraviolet radiation and comprise as essential components a combination of naturally occurring products. The compositions of the invention essentially comprise:
(1) rosemary (Rosmarinus officinalis L. extract) - 0,001 % - 10%;
(2) henna (Lawsonia inermis L. extract) - 0,001 % - 10%;
(3) melanin - 0,000005% - 5%;
the percentages being based on the total weight of the finished cosmetic composition.

In another embodiment of the invention, hair - especially dark hair - cosmetic compositions are provided which, in addition to the above-mentioned components further comprise an additional sunflower naturally occurring component as follows:
(1) rosemary (Rosmarinus officinalis L. extract) - 0,001 % - 10%;
(2) henna (Lawsonia inermis L. extract) - 0,001 % - 10%;
(3) melanin - 0,000005% - 5%;
(4) sunflower (Helianthus annuss L. extract) - 0,0001 % - 10%;
the percentages being based on the total weight of the finished cosmetic composition.

More particularly, a combination of naturally occurring products to be used in the cosmetic compositions of the present invention preferably comprises:
(1) rosemary (Rosmarinus officinalis L. extract) - 0,001 % - 0,03%;
(2) henna (Lawsonia inermis L. extract) - 0,001 % - 0,03%;
(3) melanin - 0,0001 % - 0,01 %;
(4) sunflower (Helianthus annuss L. extract) - 0,001 % - 0,005%;
the percentages being based on the total weight of the finished cosmetic composition.

The compositions of the invention are further provided with effective foaming properties, producing stable foam during application to the hair.

### Brief Description of the Drawings

Figure 1 is a graph showing photo-protection (%) of chemical structure of hair treated with compositions of the invention as a function of the time to sun exposure.
Figure 2 shows the property inherent to gloss of the compositions of the invention.
Figure 3 shows color protection to dark hair as a function of the time to sun exposure.

### Detailed Description of the Preferred Embodiments

The cosmetic compositions of the present invention are based on a combination of naturally occurring components, viz.: henna, rosemary and melanin and, optionally, sunflower. Most of the essential components of the compositions of the invention is present as extracts usually commercially available. Melanin, in turn, may be of synthetically produced.

Henna component used in the compositions of the invention is comprised of Lawsonia inermis L. extract. The main active substance of Henna is lawsonia (2-hydroxy-1,4-naphthoquinone). It is usually available in glycolic medium comprising theoxydiglycol, propylene glycol and butylene glycol. A henna-based product commercially available and particularly suitable in the compositions of this invention is EXTRAPON HENNA available from Dragoco Perfumes e Aromas Ltda.

Rosemary, also known as rosmarinus, derives from Rosmarinus officinalis L. It is usually commercially available as Rosmarinus officinalis L. extract, also in glycolic medium. A rosemary-based product commercially available and particularly suitable in the compositions of this invention is EXTRAPON ROSMARINHO available from Dragoco Perfumes e Aromas Ltda.

Melanin used in this invention is a synthetically derived product equivalent to naturally occurring melanin. It is said to be a randomly connected amorphous biopolymer. It can be synthesized from a number of precursors (L-dopa, tyrosine and other precursors known in the art). It is commonly commercially available as a solution. A melanin which is commercially available and particularly suitable in the compositions of this invention is a 10% melanin solution, known as LIPO MELANIN, available from LIPO DO BRASIL.

Sunflower used in the present invention is comprised of a sunflower (Helianthus anus L.) oil cake extract. The essential components thereof are polyphenols, in glycolic solution. A sunflower-based product particularly suitable in the present invention is HELIOGENOL, commercially available under the name SEDERMA.

The cosmetic compositions of the present invention comprise, on one side, the naturally occurring components: (1) rosemary (Rosmarinus officinalis L. extract), (2) henna (Lawsonia inermis L. extract), (3) melanin and, optionally, an additional naturally occurring component (4) sunflower (Helianthus annuss L. extract) and, on the other side, a cosmetically acceptable base. Suitable cosmetic bases both for shampoo for kids bases and shampoo for adults bases can be used.

Cosmetic bases suitable for the cosmetic compositions of this invention are well-known in the art. Cosmetic bases usually employed in cosmetic compositions may comprise, inter alia, non-ionic, anionic and amphoteric surfactants and mixtures thereof; sunscreens, conditioning agents, moisturizing agents, perfumes, chelants, viscosity agents, preservatives, color-providing agents, pearling agents, and carriers commonly used in cosmetic compositions.

The invention is not limited to specific types of bases and any bases providing an acceptable degree of eye and skin irritation as well as a suitable formulation for hair, especially dark hair, shampoo can be used according to this invention. Non-limiting examples of components usually employed in cosmetic bases for hair shampoo include: anionic surfactants such as sodium sulfate tridecyl ether, sodium sulfate lauryl ether, sodium carboxylate lauryl ether (13), ammonium lauryl sulfate, ammonium sulfate lauryl ether; amphoteric surfactants such as cocoamidopropyl betaine, disodium lauroamphodiacetate and sodium lauroamphoacetate; non-ionic surfactants such as PEG-80 sorbitan laurate, cocamide MEA Polysorbat 20, lauryl polyglycosides; moisturizers such as glycerin; antistatic agents such as polyquaternium 10 and guar hydroxypropyltrimmonium chloride; viscosity agents such as PEG-150 distearate and sodium chloride; pearling agents such as glycol mono- and/or distearate; conditioning agents such as dimeticone copolyol, polyquaternium 47, polyquaternium 7, silicone quaternium 2 pantenol succinate, PEG-14M and acrylamide and acrylamidopropyltrimmonium chloride copolymer; chelating agents such as tetrasodium EDTA and disodium EDTA; pH adjusting agents such as citric acid and sodium hydroxide; dyes such as FD&C Blue 1, D&C Red 33, D&C Violet 2; preservatives such as methylchloroisothiazolinone and methylisothiazolinone; carriers such as water and additives such as pantenol and hydrolyzed wheat protein and further additives, fragrances and usual formulating agents.

A specially preferred cosmetic base according to this invention comprises cocoamidopropyl betaine, sodium sulfate tridecyl ether, PEG-80 sorbitan laurate, disodium lauroamphodiacetate, glycerin, PEG-150 distearate, sodium carboxylate lauryl ether (13), tetrasodium EDTA, polyquaternium 10, citric acid, quaternium 15, D&C Red 33, FD&C Blue 1.

Another suitable base for use in combination with the naturally-occurring components of the compositions of the present invention is described in Brazilian Patent PI 7607893 to JOHNSON & JOHNSON.

The following examples are illustrative of specific cosmetic bases used in this invention. The particular base formulations are only illustrative and non-limiting examples of certain special types of cosmetic bases, as indicated in the tables hereinbelow. The amounts are in parts by weight based on the finished shampoo composition comprising the corresponding cosmetic base in association with the main components of the invention.

| A - Shampoo Base A | | |
|---|---|---|
| range for examples % w/w | specific example % w/w | role |
| 1.3 - 4.0 | 3.43 | anionic surfactant |
| 2.0 - 6.0 | 5.07 | amphoteric surfactant |
| 2.3 - 7.0 | 5.94 | non-ionic surfactant |
| 0.4 - 1.3 | 1.1 | amphoteric surfactant |
| 0.9 - 2.9 | 2.4 | moisturizer |
| 0.07 - 0.25 | 0.19 | antistatic agent |
| 1.0 - 3.0 | 2.54 | viscosity agent |
| 0.19 - 0.6 | 0.476 | anionic surfactant |

| B - Shampoo Base B | |
|---|---|
| % w/w | role |
| 10 | anionic surfactant |
| 2 | amphoteric surfactant |
| 0.5 | amphoteric surfactant |
| 2.0 | pearling agent |
| 0.2 | antistatic agent |
| 0.5 | conditioning agent |
| 2.0 | viscosity agent |

| C- Shampoo Base C | |
|---|---|
| % w/w | role |
| 10 | anionic surfactant |
| 2 | amphoteric surfactant |
| 1,0 | non-ionic surfactant |
| 2.0 | pearling agent |
| 0.2 | antistatic agent |
| 0.5 | conditioning agent |
| 0.4 | viscosity agent |

The following examples are illustrative of the invention and are not to be construed as limiting thereof.

### Example 1

Hair compositions were prepared using the above specific cosmetic bases with the extract preparations of the invention. A cosmetic base for shampoo compositions (base A supra) was blended with water and melanin, Lawsonia inermis L. (henna) extract and Rosmarinus officinalis L. (rosemary) extract as indicated under D below:

| D - Blend 1 | | |
|---|---|---|
| Name INCl | % w/w | role |
| melanin | 4.03 | natural UV screen |
| Lawsonia inermis L. (henna) extract | 42.70 | natural additive |
| Rosmarinus officinalis L. (rosemary) extract | 46.73 | natural additive |

### Example 2

Hair compositions were prepared using the above specific cosmetic bases with the extract preparations of the invention. A cosmetic base for shampoo compositions (base B supra) was blended with water and melanin, Lawsonia inermis L. (henna) extract and Rosmarinus officinalis L. (rosemary) extract as indicated under D below:

| D - Blend 1 | | |
|---|---|---|
| Name | % w/w | role |
| melanin | 4.03 | natural UV screen |
| Lawsonia inermis L. (henna) extract | 42.70 | natural additive |
| Rosmarinus officinalis L. (rosemary) extract | 46.73 | natural additive |

### Example 3

The same procedure as in example 2 was used for preparing a hair treatment composition using a cosmetic base with composition C above (base C).

### Example 4

Hair compositions were prepared using the above specific cosmetic bases with the extract preparations of the invention. A cosmetic base for shampoo compositions (base A supra) was blended with water and melanin, Lawsonia inermis L. (henna) extract, Rosmarinus officinalis L. (rosemary) extract and Helianthus annus L. (sunflower) extract as indicated under E below:

| E - Blend 2 | | |
|---|---|---|
| Name | % w/w | role |
| melanin | 5.4 | natural UV screen |
| Lawsonia inermis L. (henna) extract | 30.0 | natural additive |
| Rosmarinus officinalis L. (rosemary) extract | 32.2 | natural additive |
| Helianthus annus L. (sunflower) extract | 32.4 | natural additive |

### Example 5

Hair compositions were prepared using the above specific cosmetic bases with the extract preparations of the invention. A cosmetic base for shampoo compositions (base B supra) was blended with water and melanin, Lawsonia inermis L. extract, Rosmarinus officinalis L. (rosemary) extract and Helianthus annus L. (sunflower) extract as indicated under E below:

| E - Blend 2 | | |
|---|---|---|
| Name | % w/w | role |
| melanin | 5.4 | natural UV screen |
| Lawsonia inermis L. (henna) extract | 30.0 | natural additive |
| Rosmarinus officinalis L. (rosemary) extract | 32.2 | natural additive |
| Helianthus annus L. (sunflower) extract | 32.4 | natural additive |

### Example 6

Hair compositions were prepared using the above specific cosmetic bases with the extract preparations of the invention. A cosmetic base for shampoo compositions (base C supra) was blended with water and melanin, Lawsonia inermis L. extract, Rosmarinus officinalis L. (rosemary) extract and Helianthus annus L. (sunflower) extract as indicated under E below:

| E - Blend 2 | | |
|---|---|---|
| Name | % w/w | role |
| melanin | 5.4 | natural UV screen |
| Lawsonia inermis L. (henna) extract | 30.0 | natural additive |
| Rosmarinus officinalis L. (rosemary) extract | 32.2 | natural additive |
| Helianthus annus L. (sunflower) extract | 32.4 | natural additive |

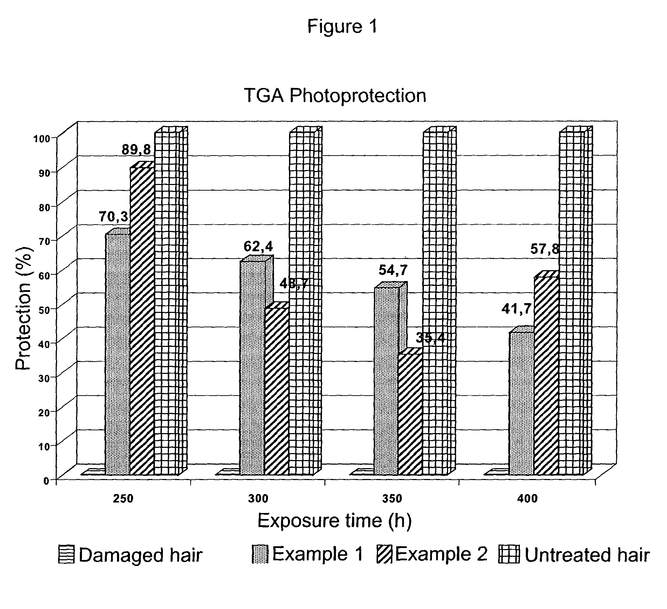

Figure 1 is a bar graph illustrating photo-protection (%) of hair treated with compositions of the invention as a function of the time to sun exposure. Two samples of hair treated with compositions of this invention were evaluated compared to a sample of fully damaged hair and a sample of untreated hair, free from any damages. Control samples (extremities) are samples of fully damaged hair (0% protection) and fully untreated hair where full protection (100%) is presumed. Evaluation was carried out by thermogravimetric analysis (TGA) whereby a change in weight of a hair sample as a function of temperature and time is observed. Such technique allows to ascertain protection of hair chemical structure. The experiment was carried out with hair fibers about 3 mm thick in samples with a total weight fibers of about 10 mg.

The samples were subjected to a temperature raise from 150 to 600°C at a 5°C/min rate.

The assay was conducted with a set of four samples and the results are plotted on figure 1. Samples of hair treated with the compositions of this invention were evaluated in relation to control samples (fully damaged hair and fully untreated hair). The results of photo-protection versus time of exposure (h) are reported on figure 1. The results show photo-protection efficacy provided by two shampoo formulations according to this invention (formulations from examples 1 and 4).

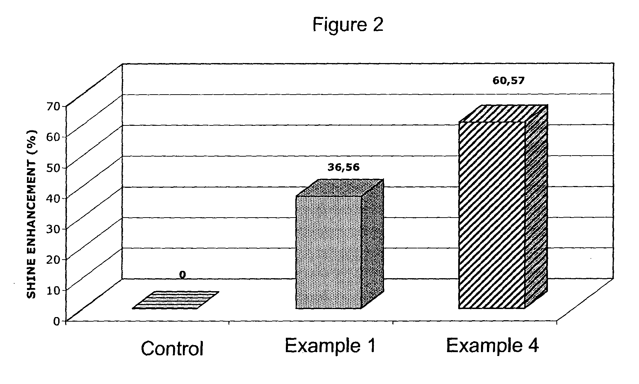

Figure 2 illustrates the property inherent to gloss provided by cosmetic compositions of the present invention. Hair samples were treated with compositions of this invention and evaluated for gloss.

For evaluating gloss, samples of curls were first photo-damaged upon exposure for 150 hours in a xenon arc simulator of sunlight for giving photo-damaged and dried hair. The samples of damaged hair were then subjected to a treatment with a 1:1 ethanol/chloroform solution followed by treatment with a 3% ammonium lauryl sulfate (ALS). Then the treated samples were rinsed with water for 30 seconds at 38°C and 0.5 l/min.

The rinsed samples were then treated with compositions of this invention for 30 seconds, followed by rinsing for the next 30 seconds. Evaluation for gloss was carried out using a GLOSSMETER (Rhopoint™). Gloss performance compared to a control shampoo-free and subjected just to one washing step with 3% ALS is shown in figure 2. Compositions used for each assay were those from examples 1 and 4, respectively, as shown in figure 2. On the average, about 36.56% and 60.57% gloss increase were reached compared to the control. Significant performance as to gloss is obtained with the compositions of the present invention. Enhanced gloss was obtained with compositions of this invention containing sunflower (60.57%) (composition from example 4).

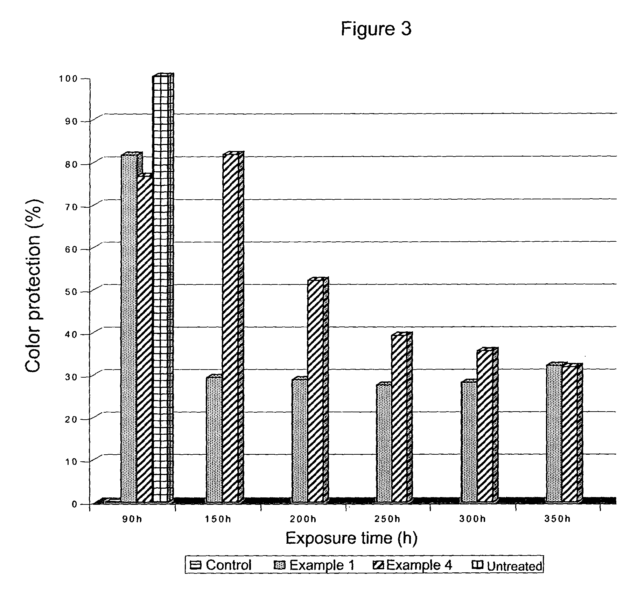

Figure 3 shows the efficacy of the compositions used in examples 1 and 4 regarding color protection of dark hair exposed to solar radiation. Protection is demonstrated by a measure of coloration of hair fibers. Such coloration is monitored through parameter L of the color system L*a*b CIE (Comission Internacionale de l'Eclairage, 1976) using a colorimeter. As with the thermogravimetric analysis, two samples of hair treated with compositions of the invention were evaluated in comparison with a sample of hair fully damaged and a sample of untreated hair, free from any discoloration. Control samples (extremities) are samples of fully damaged (100% discolored) hair and untreated hair with no discoloration at all.

The results shown in figure 1 show the ability of the compositions of this invention to provide protection of the hair chemical structure against solar photo-degradation. Figure 2 shows the significant increase in gloss provided by the compositions.

Figure 3 shows color protection for dark hair due to a treatment with compositions of the invention.

## Claims

1. A cosmetic composition wherein said composition comprises:
(1) Rosemary (Rosmarinus officinalis L. extract) - 0,001 % - 10%;
(2) Henna (Lawsonia inermis L. extract) - 0,001 % - 10%
(3) Melanin - 0,000005% - 5%;
the percentages being based on the total weight of the finished cosmetic composition.

2. A cosmetic composition according to claim 1, wherein said composition comprises:
(1) Rosemary (Rosmarinus officinalis L. extract) - 0,001 % - 10%;
(2) Henna (Lawsonia inermis L. extract) - 0,001 % - 10%
(3) Melanin - 0,000005% - 5%;
(4) Sunflower (Helianthus annuss L. extract) - 0,0001 % - 10%;
the percentages being based on the total weight of the finished cosmetic composition.

3. A cosmetic composition according to claim 1 or 2, wherein said composition comprises:
(1) Rosemary (Rosmarinus officinalis L. extract) - 0,001 % - 0,03%;
(2) Henna (Lawsonia inermis L. extract) - 0,001 % - 0,03%;
(3) Melanin - 0,0001 % - 0,01 %;
(4) Sunflower (Helianthus annuss L. extract) - 0,001 % - 0,005%;
the percentages being based on the total weight of the finished cosmetic composition.

4. A cosmetic composition according to claim 1, 2 or 3, further comprising a cosmetically acceptable base composition for shampoo.

5. A cosmetic composition according to claim 1, 2, 3 or 4, wherein said base comprises non-ionic, anionic, amphoteric surfactants and mixtures thereof; sunscreens, conditioning agents, moisturizing agents, perfumes, chelants, viscosity agents, preservatives, color-providing agents, pearling agents, and carriers commonly used in formulating cosmetic compositions.

6. A cosmetic composition according to claim 1, 2, 3, 4 or 5, wherein said base comprises anionic surfactants such as sodium sulfate tridecyl ether, sodium sulfate lauryl ether, sodium carboxylate lauryl ether (13), ammonium lauryl sulfate, ammonium sulfate lauryl ether; amphoteric surfactants such as cocoamidopropyl betaine, disodium lauroamphodiacetate and sodium lauroamphoacetate; non-ionic surfactants such as PEG-80 sorbitan laurate, cocamide MEA Polysorbat 20, lauryl polyglycosides; moisturizers such as glycerin; antistatic agents such as polyquaternium 10 and guar hydroxypropyltrimmonium chloride; viscosity agents such as PEG-150 distearate and sodium chloride; pearling agents such as glycol mono- and/or distearate; conditioning agents such as dimeticone copolyol, polyquaternium 47, polyquaternium 7, silicone quaternium 2 pantenol succinate, PEG-14M and acrylamide and acrylamidopropyltrimmonium chloride copolymer; chelating agents such as tetrasodium EDTA and disodium EDTA; pH adjusting agents such as citric acid and sodium hydroxide; dyes such as FD&C Blue 1, D&C Red 33, D&C Violet 2; preservatives such as methylchloroisothiazolinone and methylisothiazolinone; carriers such as water and additives such as pantenol and hydrolyzed wheat protein and further additives, fragrances and usual formulating agents.

7. A cosmetic composition according to claim 1, 2, 3, 4 or 5, wherein said base comprises cocoamidopropyl betaine, sodium sulfate tridecyl ether, PEG 80 sorbitan laurate, disodium lauroamphodiacetate, glycerin, PEG-150 distearate, sodium carboxylate lauryl ether (13), tetrasodium EDTA, polyquaternium 10, citric acid, quaternium 15, D&C Red 33, FD&C Blue 1.

8. A concentrated composition suitable for use in cosmetic compositions, wherein said composition comprises:
(1) rosemary (Rosmarinus officinalis L. extract) - 0,001 % - 10%;
(2) henna (Lawsonia inermis L. extract) - 0,001 % - 10%;
(3) melanin - 0,000005% - 5%; the percentages being based on the total weight of the finished cosmetic composition.

9. A concentrated composition according to claim 6, wherein said composition comprises:
(1) rosemary (Rosmarinus officinalis L. extract) - 0,001 % - 10%;
(2) henna (Lawsonia inermis L. extract) - 0,001 % - 10%;
(3) melanin - 0,000005% - 5%;
(4) sunflower (Helianthus annuss L. extract) - 0,0001 % - 10%;
the percentages being based on the total weight of the finished cosmetic composition.
